# EUROPEAN PATENT APPLICATION

(11) **EP 4 846 925 A1**
(43) Date of publication of application: **02.12.2026**
(21) Application number: 25178811.3
(22) Date of filing: 26.05.2025
(51) Int. Cl.: A61B 5/00, A61B 5/087

(54) **TRANSDUCER UNIT, AIRWAY ADAPTER AND GAS ANALYZER**

(71) Applicant: Sensirion AG, 8712 Stäfa (CH)
(72) Inventor: ZIPKES, Christoph, 8712 Stäfa (CH); MERZ, Matthias, 8712 Stäfa (CH)
(74) Representative: Detken, Andreas

(57) **Abstract**

A transducer unit for a gas analyzer is disclosed, comprising a housing comprising a base (210) and first and second lateral wall structures (220, 230) extending away from the base (210) in a coupling direction (C) and being arranged at a distance with respect to a lateral direction (y) that is transverse to the coupling direction (C), defining a receiving space between them for receiving an airway adapter. Each lateral wall structure (220, 230) has a guide surface (221, 231) that faces the receiving space. Optical windows (228, 238) are formed in the first lateral wall structure (220) at the guide surfaces (221, 231). A radiation emitter (251) is received in the housing to emit infrared radiation (253) towards the receiving space, and a radiation detector (252) is received in the housing to detect a portion of the infrared radiation (253) that has traversed the receiving space (201). At least one of the optical windows (228, 238) that has a protruding window portion protruding from the associated guide surface (221, 231) inwardly towards the receiving space. Also disclosed is an airway adapter for use with the transducer unit.

## Description

### TECHNICAL FIELD

The present invention relates to a transducer unit for a gas analyzer, an airway adapter for use with the transducer unit, and to a gas analyzer comprising the transducer unit and the airway adapter.

### PRIOR ART

In a clinical context it may be crucial to monitor the CO₂ level in the exhaled air of a patient, in particular, the end-tidal CO₂ level (ETCO₂). ETCO₂ reflects the efficiency with which CO₂ is carried in the blood back to the lungs and exhaled.

Non-invasive methods for ETCO₂ measurement include capnometry and capnography. Capnometry provides a numerical value for ETCO₂. In contrast, capnography delivers a more comprehensive measurement that can be displayed in both graphical (waveform) and numerical form. Capnometry or capnography devices are typically configured as either sidestream or mainstream. In a sidestream configuration, the monitoring device with the CO₂ sensor is at a distance from the patient. The exhaled CO₂ is diverted from the airway into the device via a sampling tube, which is attached to the breathing circuit fitted to the patient. In the case of a mainstream configuration, the CO₂ sensor and a sampling cell are integrated into a small device that connects directly at the airway. The sampling cell can be configured as an airway adapter, having connectors for connecting one end to a patient interface such an endotracheal tube and the other end to a ventilator. Capnometry or capnography devices are available as hand-held portable devices or as a module or component integrated into other medical equipment, such as defibrillators, anesthesiology machines, and patient-monitoring systems.

In order to determine the level of CO₂ in the exhaled breath of the patient, it is known to pass infrared radiation through a breath sample and to measure the absorption of the infrared radiation. A large variety of devices for determining the level of CO₂ in a breath sample based on this principle have been proposed in the prior art.

US4859858A discloses a portable, hand-held gas analyzer designed to monitor the concentration of carbon dioxide in the exhalations of a medical patient. The gas analyzer comprises a transducer unit and an airway adapter. The airway adapter is a disposable one-piece unit. It is employed to connect, e.g., an endotracheal tube inserted into the patient's trachea to the plumbing of a mechanical ventilator. The airway adapter confines the expired gases to a flow path with a precise transverse dimension and furnishes an optical path between an infrared radiation emitter and an infrared radiation detector in the transducer unit. Apertures are formed in a central section of the airway adapter to allow infrared radiation to enter and exit the airway adapter. To keep the gases flowing through the airway adapter from escaping through the apertures, the apertures are sealed by sapphire windows.

US5067492A discloses an airway adapter having optical windows formed of the same material as the adapter body, e.g., polyethylene or polypropylene.

The optical windows of the airway adapter are generally thin compared to the wall sections of the airway adapter in which the apertures are formed to minimize unwanted absorption of infrared light. In the above prior-art examples, the optical windows are flush with an exterior surface of the airway adapter. However, it may be desirable that the windows are flush with an interior surface that delimits a sampling passage for the breath sample. On the one hand, condensation is reduced in this manner because the flow of the breath sample overflows the windows. On the other hand, it may be easier to manufacture the windows in one piece with a base body of the airway adapter by injection molding if the windows are flush with an interior surface of the airway adapter and set back from an exterior surface.

The transducer unit typically also has optical windows to allow infrared radiation to pass from the transducer unit into the airway adapter and vice versa. When the transducer unit is coupled to the airway adapter, each window of the transducer unit is typically located at a certain distance to an adjacent window of the airway adapter. Due to this distance, a dead space is formed between each window of the transducer unit and the adjacent window of the airway adapter. When the transducer unit is connected to the airway adapter, CO₂ can be trapped between these windows. This can lead to measurement errors when CO₂ concentration in the sampling passage is measured. It may therefore be necessary to put the airway adapter with the transducer unit coupled thereto away from any potential sources of CO₂ such as exhaled breath and to wait a considerable amount of time until the CO₂ in the dead space has diffused away from the dead space before any baseline measurements are initiated.

### SUMMARY OF THE INVENTION

It is an object of the present invention to reduce the effects of CO₂ trapped in the dead space between a window of the transducer unit and a window of the airway adapter.

This object is achieved with a transducer unit as defined in claim 1. Further embodiments are laid down in the dependent claims.

A transducer unit for a gas analyzer is provided, comprising:
a housing comprising a base and first and second lateral wall structures extending away from the base in a coupling direction and being arranged at a distance to each other with respect to a lateral direction that is transverse to the coupling direction, the first and second lateral wall structures defining a receiving space between them for receiving an airway adapter, the receiving space being open in the coupling direction;
a radiation emitter; and
a radiation detector;
wherein the first lateral wall structure has a first guide surface that faces the receiving space,
wherein a first optical window is provided in the first lateral wall structure at the first guide surface,
wherein the radiation emitter is received in the housing to emit infrared radiation towards the receiving space through the first optical window ,
wherein the second lateral wall structure has a second guide surface that faces the receiving space,
wherein a second optical window is provided in the second lateral wall structure at the second guide surface,
wherein the radiation detector is received in the housing to detect a portion of the infrared radiation that has traversed the receiving space and the second optical window along the lateral direction, and
wherein at least one of the first and second optical windows has a protruding window portion that protrudes from the first or second guide surface, respectively, inwardly towards the receiving space.

By providing the transducer unit with at least one inwardly protruding window portion, the distance between the protruding window portion and a corresponding window of the airway adapter is reduced. Thereby the dead space between these windows is reduced. Thereby, reliability of the measurements that are carried out with the gas analyzer is improved. Preferably, each of the first and second optical windows has a protruding window portion.

A direction is understood to extend "transversely" to another direction if the directions include an angle in the range of 45° to 135°, preferably 60° to 120°, more preferably 90° (in the latter case, the directions are orthogonal to each other).

In some embodiments, the protruding window portion is convexly curved. In particular, the protruding window portion may be convexly curved both along the coupling direction and along a longitudinal direction that extends transversely to both the coupling direction and the lateral direction. The protruding window portion thus forms a converging lens for shaping the infrared radiation. In other embodiments, the protruding window portion may be cylindrical.

In some embodiments, the optical window having the protruding window portion is spherical. It may be formed by a ball of an infrared-transparent material. Spherical windows can be manufactured in a particularly efficient manner and do not require precise alignment.

The transducer unit may further comprise a window support arranged adjacent to the protruding window portion with respect to the coupling direction. The window support protrudes inwardly from the associated guide surface towards the receiving space. It is preferably formed integrally (in one piece) with the housing portion that forms the associated guide surface. In order to efficiently protect the protruding window portion from damage when the transducer unit is coupled with the airway adapter, the window support preferably protrudes inwardly from the associated guide surface towards the receiving space at least by the same amount as the protruding window portion. For similar reasons, it preferably has a longitudinal dimension, measured along the longitudinal direction, that at least corresponds to a maximum longitudinal dimension of the protruding window portion, measured along the longitudinal direction.

In some embodiments, the window support extends on both sides of the protruding window portion with respect to the coupling direction, thus providing additional mechanical strength to the housing at the respective guide surface.

Also disclosed is an airway adapter for use with the transducer unit disclosed herein. The airway adapter comprises:
a base body defining a sampling passage for a gas to be analyzed, the sampling passage extending through the base body along a longitudinal direction, the base body having a first side surface on a first lateral side of the base body and a second side surface on an opposing second lateral side of the base body with respect to a center plane that contains the longitudinal direction and passes centrally through the sampling passage;
a first optical window at the first lateral side of the base body and a second optical window at the second lateral side of the base body to allow infrared radiation to enter and exit the sampling passage transversely to the longitudinal direction; ; and
a first protrusion-receiving groove formed in the first side surface of the base body, the first protrusion-receiving groove extending in a coupling direction that extends transversely both to the longitudinal direction and to a lateral direction, the lateral direction being perpendicular to the center plane, the first protrusion-receiving groove being a blind groove extending between an open end located at a peripheral boundary of the first side surface and a closed end positioned within the first side surface,
wherein the first optical window is arranged in the first protrusion-receiving groove.

The first protrusion-receiving groove is configured to receive a protruding window portion and/or a window support of the transducer unit when the transducer unit is coupled to the airway adapter. By providing a protrusion-receiving groove, which may be rather narrow, instead of thinning the entire sidewall of the airway adapter in which the first optical window of the airway adapter is formed, mechanical stability of the airway adapter can be ensured.

The first optical window of the airway adapter may be flush with a bottom surface of the first protrusion-receiving groove or may be set back from the bottom surface of the first protrusion-receiving groove with respect to the lateral direction.

The first protrusion-receiving groove is preferably not wider than necessary so as not to compromise stability of the airway adapter. In particular, the first protrusion-receiving groove may have a width, measured along the longitudinal direction, which is no greater than 150% of the diameter of the first optical window of the airway adapter, preferably no greater than 120% of the diameter of said first optical window. In some embodiments, the width of the first groove may correspond to the diameter of said first optical window.

In order to provide guidance for the protruding window portion and/or window support of the transducer unit, the first protrusion-receiving groove may have a length, measured along the coupling direction, which is at least 150% of the diameter of the first optical window of the airway adapter.

The first protrusion-receiving groove may have a considerable depth measured along the lateral direction to effectively reduce the dead space. In particular, said depth may be at least 50% of the remaining wall thickness between the first protrusion-receiving groove and the sampling passage (expressed differently, at least 50% of the distance of the bottom surface of the first protrusion-receiving groove to the sampling passage), measured along the lateral direction.

The base body may have a second protrusion-receiving groove formed in the second side surface of the base body, the second protrusion-receiving groove extending parallel to the first groove. Like the first protrusion-receiving groove, also the second protrusion-receiving groove is preferably a blind groove extending between an open end located at a peripheral boundary of the second side surface and a closed end positioned within the second side surface. The second optical window of the airway adapter may be arranged in the second protrusion-receiving groove. All considerations with respect to the dimensions of the first protrusion-receiving groove also apply for the second protrusion-receiving groove.

Each of the first and second optical windows of the airway adapter may be formed integrally with the base body, being formed by a thinned sidewall portion of the base body. The first and second optical windows of the airway adapter may be aligned along the lateral direction such that their centers lie on an imaginary straight line that extends along the lateral direction, or they may be offset relative to each other in the coupling direction such that their centers are offset from each other. In particular, the centers of the first and second optical windows of the airway adapter may be aligned along a direction that has a component along the coupling direction and deviates from the lateral direction by not more than 45°, preferably by not more than 30°, in particular, not more than 20°. Likewise, the centers of the first and second optical windows of the transducer unit may be aligned along the lateral direction, or they may be offset with respect to the lateral direction, being aligned along a direction that has a component along the coupling direction and deviates from the lateral direction by not more than 45°, preferably by not more than 30°, in particular, not more than 20°. An optical axis extending between the radiation emitter and the radiation detector of the transducer unit may pass through the centers of the optical windows of the airway adapter and of the transducer unit or may intersect these windows slightly off-center.

Also disclosed is a gas analyzer comprising the transducer unit disclosed herein and the airway adapter disclosed herein. The transducer unit is configured to be coupled to the airway adapter along the coupling direction. The first protrusion-receiving groove receives the at least one protruding window portion of the transducer unit when the transducer unit is coupled to the airway adapter.

The protrusion-receiving groove preferably has a width, measured along the longitudinal direction, that essentially corresponds to an outer dimension of the protruding window portion or of an associated window support, measured along the longitudinal direction.

The first guide surface preferably abuts against the first side surface and the second guide surface abuts against the second side surface when the transducer unit is coupled to the airway adapter. Thereby, the dead space is kept small, and these surfaces define the relative position of the transducer unit and the airway adapter with respect to the lateral direction.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows an airway adapter according to a first embodiment in a perspective view;
- Fig. 2: shows the airway adapter of Fig. 1 in another perspective view;
- Fig. 3: shows the airway adapter of Fig. 1 in a left side view;
- Fig. 4: shows the airway adapter of Fig. 1 in a bottom view;
- Fig. 5: shows a transducer unit according to a first embodiment in a perspective view;
- Fig. 6: shows the transducer unit of Fig. 5 in another perspective view;
- Fig. 7: shows the transducer unit of Fig. 5 in a cross section in the same plane as for the airway adapter in Fig. 8;
- Fig. 8: shows the airway adapter of Fig. 1 in a cross section in plane S1-S1 indicated in Fig. 3;
- Fig. 9: shows the airway adapter of Fig. 1 and the transducer unit of Fig. 5 in a coupled state in a cross section in the same plane as in Fig 8;
- Fig. 10: shows the transducer unit of Fig. 5 in a cross section in the same plane as for the airway adapter in Fig. 11;
- Fig. 11: shows the airway adapter of Fig. 1 in a cross section in plane S2-S2 indicated in Fig. 3;
- Fig. 12: shows the airway adapter of Fig. 1 and the transducer unit of Fig. 5 in a coupled state in a cross section in the same plane as in Fig. 11;
- Fig. 13: shows an airway adapter according to a second embodiment in a perspective view;
- Fig. 14: shows the airway adapter of Fig. 13 in another perspective view;
- Fig. 15: shows the airway adapter of Fig. 13 in a left side view;
- Fig. 16: shows the airway adapter of Fig. 13 in a bottom view;
- Fig. 17: shows a transducer unit according to a second embodiment in a perspective view;
- Fig. 18: shows the airway adapter of Fig. 13 and the transducer unit of Fig. 17 in a coupled state in a cross section in plane S3-S3 indicated in Fig. 15;
- Fig. 19: shows a transducer unit according to a third embodiment in a perspective view;
- Fig. 20: shows an airway adapter according to a third embodiment in a perspective view;
- Fig. 21: shows the airway adapter of Fig. 20 in a top view;
- Fig. 22: shows the airway adapter of Fig. 20 and the transducer unit of Fig. 19 in a coupled state in a cross section in plane S4-S4 indicated in Fig. 21;
- Fig. 23: shows a transducer unit according to a fourth embodiment in a perspective view;
- Fig. 24: shows an airway adapter according to a fourth embodiment in a perspective view;
- Fig. 25: shows the airway adapter of Fig. 24 and the transducer unit of Fig. 23 in a coupled state in a cross section;
- Fig. 26: shows a transducer unit according to a fifth embodiment in a perspective view;
- Fig. 27: shows a transducer unit according to a sixth embodiment in a perspective view;
- Fig. 28: shows an airway adapter according to a fifth and sixth embodiment in a perspective view;
- Fig. 29: shows the airway adapter of Fig. 28 and the transducer unit of Fig. 26 in a coupled state in a cross section;
- Fig. 30: shows the airway adapter of Fig. 28 and the transducer unit of Fig. 27 in a coupled state in a cross section;
- Fig. 31: shows a transducer unit according to a seventh embodiment in a bottom view;
- Fig. 32: shows an airway adapter according to a seventh embodiment in a perspective view;
- Fig. 33: shows the airway adapter of Fig. 32 in a top view;
- Fig. 34: shows the airway adapter of Fig. 32 in a front view along viewing direction z;
- Fig. 35: shows the airway adapter of Fig. 32 in a cross section in plane S5-S5 indicated in Fig. 33;
- Fig. 36: shows the airway adapter of Fig. 32 in a cross section in plane S6-S6 indicated in Fig. 33;
- Fig. 37: shows the airway adapter of Fig. 32 in a cross section in plane S7-S7 indicated in Fig. 33;
- Fig. 38: shows the airway adapter of Fig. 32 in a perspective sectional view in plane S7-S7 indicated in Fig. 33;
- Fig. 39: shows the airway adapter of Fig. 32 in a longitudinal section in plane S8-S8 indicated in Fig. 33; and
- Fig. 40: shows the airway adapter of Fig. 32 in a cross section in plane S9-S9 indicated in Fig. 33; and
- Fig. 41: shows an airway adapter according to an eight embodiment and a corresponding transducer unit in a coupled state in a cross section.

### DESCRIPTION OF PREFERRED EMBODIMENTS

### First embodiment: setup of airway adapter

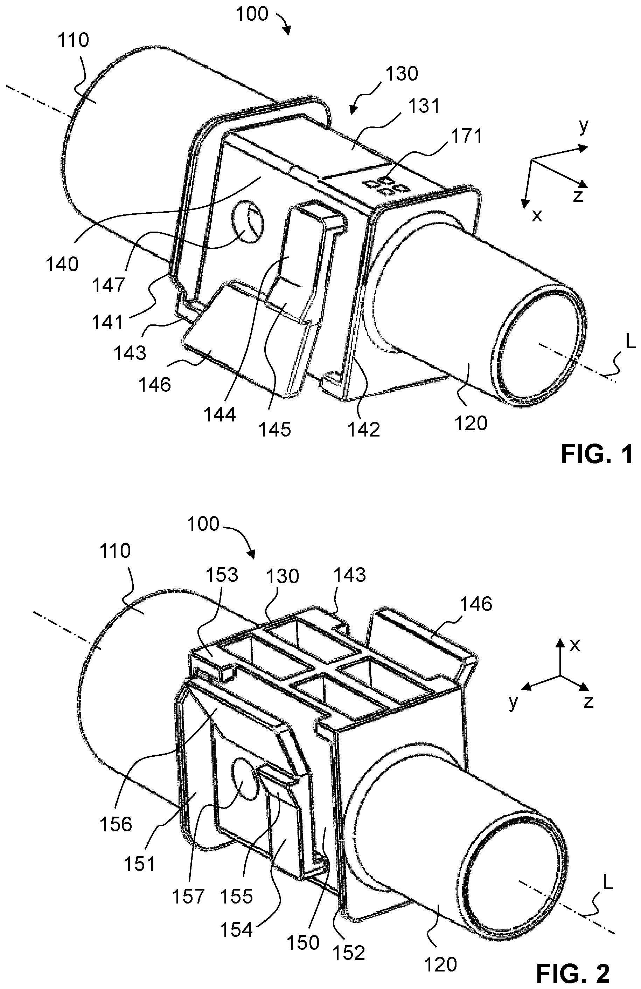

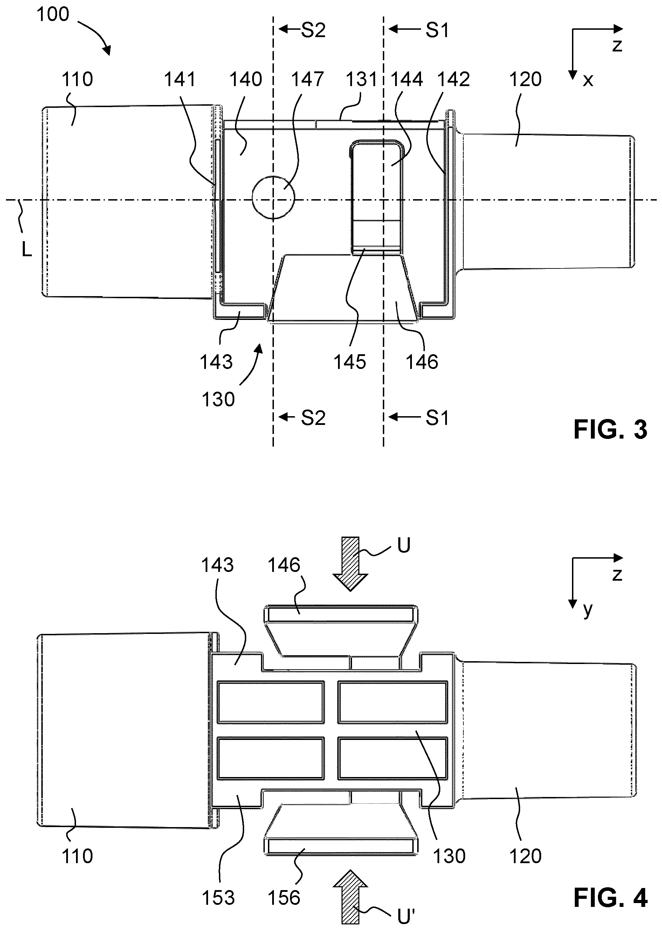

Figures 1 to 4, 8 and 11 illustrate, in various views, a first exemplary embodiment of an airway adapter 100.

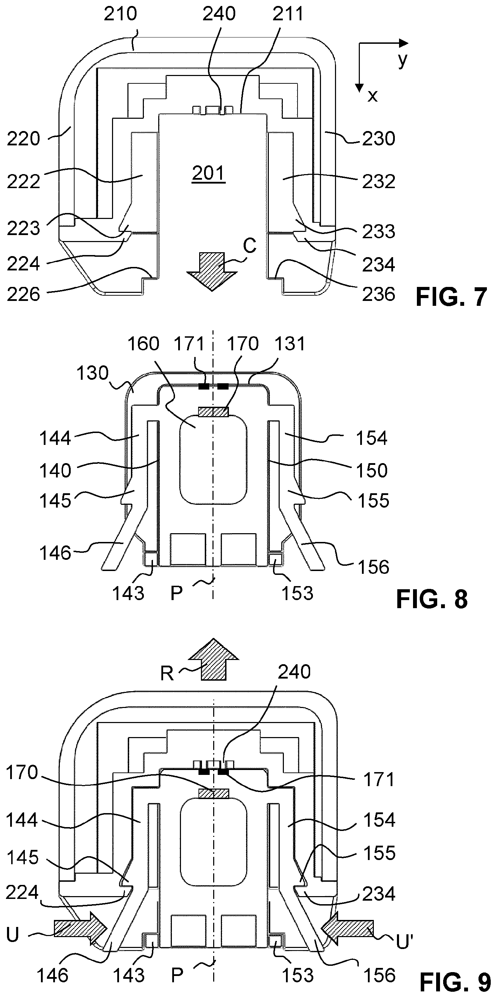

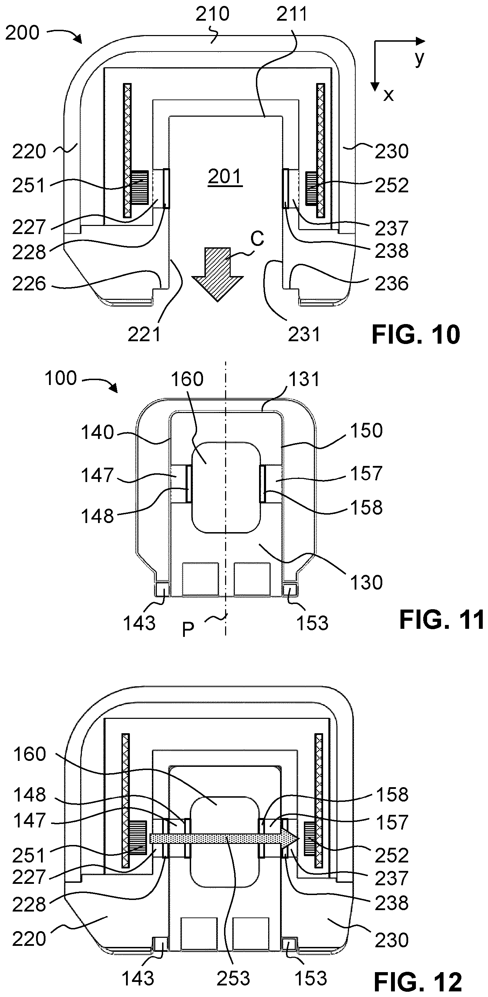

The airway adapter 100 comprises a generally parallelepipedal base body 130 defining a sampling passage 160 (see Figs. 8 and 11) extending from end-to-end therethrough. The airway adapter 100 further comprises two hollow end sections 110 and 120 arranged at mutually opposite ends of the base body 130, which may be formed in one piece with at least a portion of the base body 130. The end sections 110 and 120 are axially aligned with the base body 130 along a common longitudinal centerline L. The base body 130 forms a center section of the airway adapter 100. Longitudinal through passages are formed in the end sections 110, 120, the through passages cooperating with the sampling passage 160 through the base body 130 to form a single, continuous, elongated flow channel extending from end-to-end through the airway adapter 100. The through passages in the end sections 110, 120 thus serve as an inlet and an outlet for the sampling passage 160. The sampling passage 160 forms a cavity in the base body 130 which confines a sample composed of one or more gases to a defined flow path. As will be explained in more detail below, the sampling passage 160 is traversed along a lateral direction by an optical path between an infrared radiation emitter and an infrared radiation detector when the airway adapter 100 is coupled to a corresponding transducer unit.

Mutually orthogonal directions x, y, z can be defined as follows: The z direction is the direction of elongated sampling passage 160 from the first end section 110 to the second end section 120. The z direction is also referred to as the longitudinal direction. The x direction corresponds to the direction along which a transducer unit is coupled to the airway adapter. The x direction is also referred to as the coupling direction C (see Fig. 7) or the vertical direction, although this direction does not need to be vertical with respect to the direction of gravity. The y direction corresponds to the direction in which infrared radiation is passed through the sampling passage 160. The y direction is also referred to as the lateral direction. Directions x, y z form a right-handed coordinate system.

A vertical center plane P (see Fig. 8) extends perpendicular to the y direction through the center of the sampling passage 160. The center plane P contains the centerline L.

The base body 130 has a top surface 131 and two planar side surfaces 140, 150 on opposite sides of the base body 130 with respect to the center plane P. The side surfaces 140, 150 are parallel to each other and extend parallel to the center plane P. With respect to the negative z direction, side surface 140 is delimited towards the first end section 110 by a first protruding guide flange 141. Likewise, with respect to the positive z direction, side surface 140 is delimited towards the second end section 110 by a second protruding guide flange 142. The guide flanges 141, 142 extend parallel to each other and perpendicular to the center plane P. Similarly, side surface 150 is delimited towards the first and second end sections 110, 120, respectively, by parallel guide flanges 151, 152. With respect to the positive x direction, each side surface 140, 150 is delimited by a protruding stop flange 143, 153, respectively.

Lateral apertures 147, 157 are formed in the base body 130 at the side surfaces 140, 150. The apertures 147, 157 are aligned along the y direction. As best seen in Fig. 11, each aperture 147, 148 is closed towards the sampling passage 160 by an optical window 148, 158. The windows are at least partially transparent for infrared radiation. Each window may be formed integrally (in one piece) with the base body 130 as a thinned sidewall portion of the base body 130. In this case, the apertures 147, 157 may also be referred to as recesses, since they do not fully traverse the corresponding side wall of the base body, ending at the respective window 148, 158. In other embodiments, the windows may be manufactured separately and mounted in the respective aperture, e.g., by gluing. The windows 148, 158 are preferably flush with the inner surface of the base body 130 that delimits the sampling passage 160 and are set back from the side surfaces 140, 150.

On a first lateral side of the base body 130 with respect to the center plane P, a first locking lever 144 is provided on the base body 130. The first locking lever 144 takes the form of a spring arm having a proximal end and a distal end. The proximal end of the spring arm is fixedly connected to the base body 130 near the top end of side surface 140, the spring arm being integrally formed with the base body 130. The spring arm extends downwardly parallel to the x direction at a distance from the side surface 140. The spring arm is flexible such that it can bend inwards towards the center plane P against an elastic restoring force that acts away from the center plane P. A first locking structure 145 in the form of a hook- or barb-like locking lug is formed at the outside of the first locking lever 144 near its free distal end. The locking lug has a sliding surface and a locking surface. The sliding surface extends in a plane that is inclined relative to the center plane P, having a surface normal with a component outwardly away from the center plane P and a component in the negative x direction. In the present example, the angle of inclination of the sliding surface relative to the center plane P (or, equivalently, the angle between the surface normal of the sliding surface and the y direction) is approximately 30° but may deviate from this value and may for example be in the range of 15° to 45°. The locking surface extends in a plane whose surface normal points in the positive x direction but may instead be slightly inclined inwardly or outwardly with respect to this plane, e.g. by an angle between -15° and +15°.

A first handle 146 having a flat wing-like shape extends downwardly and outwardly from the free distal end of the first locking lever 144. The handle 146 may also referred to as a gripping flange or gripping wing. At its outside, facing away from the center plane P, the handle 146 has a lateral gripping surface. The handle 146 is tilted relative to the center plane P such that the gripping surface is inclined, its surface normal having both a laterally outwardly pointing component away from the center plane P and an upwardly pointing component along the negative x direction. The handle 146 has a larger dimension along the longitudinal direction z than the locking lever 144, in the present example by about a factor of three.

An identically shaped second locking lever 154, an identically shaped second locking structure 155 and an identically shaped second handle 156 are provided on the opposite lateral side of the base body 130 with respect to the center plane P. The locking levers 144, 154, the locking lugs 145, 155 and the handles 146, 156 on both sides of the base body 130 are configured and arranged in a mirror-symmetric manner to one another with respect to the center plane P.

As best seen in Figures 8 and 9, the airway adapter 100 may optionally comprise an electrically operated sensor 170 for determining at least one additional parameter of the gas inside the sampling passage 160 in addition to the CO₂ concentration. For example, the sensor 170 may be a microthermal flow rate sensor for determining flow rate information, a gas concentration sensor for determining additional composition information (e.g., the concentration of molecular oxygen (O₂) or water vapor (humidity), nitric oxides, or anesthetic agents such as halothane, enflurane, isoflurane, sevoflurane and desflurane) and/or a physical sensor for determining a physical gas property such as temperature and/or pressure of the gas in the sampling passage 160. Contact pads 171 are formed at the top surface 131 of the base body 130 of the airway adapter 100 to establish an electrical connection to the sensor 170.

### First embodiment: setup of transducer unit

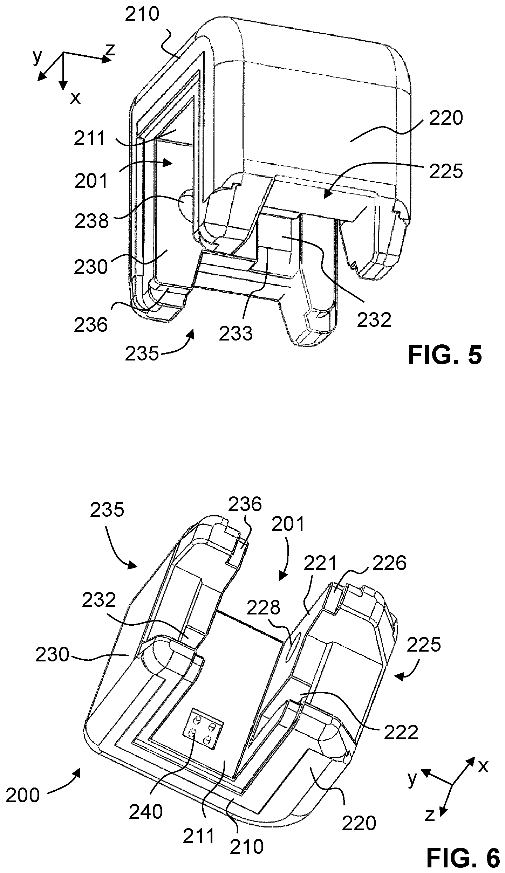

Figures 5, 6, 7 and 10 illustrate a transducer unit 200 configured to be connected with the airway adapter 100. The transducer unit 200 may also be referred to a sensor unit, sensor head or transducer head or as a U clip because of its U shape.

As best visible in Figures 5 and 6, the transducer unit 200 comprises a housing comprising a base 210 defining a horizontal support surface 211 and two mutually opposing lateral wall structures 220, 230 extending downwardly from the base 210 along the positive x direction. Between the lateral wall structures 220, 230, a receiving space 201 (which may also be referred to as a receiving recess) for receiving the airway adapter 100 is formed. Each lateral wall structure 220, 230 has a planar guide surface 221, 231 facing the receiving space 201. Stop surfaces 226, 236 facing in the positive x direction are formed at each lateral wall structure 220, 230. Deep gripping recesses 225, 235 are formed at the end of each lateral wall structure 220, 230 that faces away from the base 210. Spring-loaded contact pins 240 (also known as pogo pins) are provided in the base 210 at the support surface 211.

As best visible in Figures 5, 6 and 7, vertical guide grooves 222, 232 extending parallel to the x direction are formed in the guide surfaces 221, 231 of the lateral wall structures 220, 230. The guide grooves 222, 232 are blind grooves extending between a closed end near the support surface 211 and an open end at a peripheral boundary of the respective guide surface 221, 231. They are thus open in the positive x direction. Near its open end, each guide groove 222, 232 deepens to form a locking recess 223, 233. Each locking recess is delimited towards the positive x direction by an inwardly projecting web, which forms a locking stop 224, 234.

The housing of the transducer unit 200 defines a hollow interior in which various electronic components are received. As best visible in Fig. 10, apertures 227, 237 are formed in the lateral wall structures 220, 230 at the guide surfaces 221, 231 between the receiving space 201 and the hollow interior of the transducer unit 200. The apertures 227, 237 are aligned along the y direction. They are sealed by optical windows 228, 238 that are at least partially transparent for infrared radiation. Each window may be formed integrally (in one piece) with the respective guide surface of the corresponding lateral wall structure 220, 230 as a thinned sidewall portion of the lateral wall structure (in this case, also the apertures 227, 237 may be referred to as recesses), or each window may be manufactured separately and mounted in the respective aperture, e.g., by gluing. The windows 228, 238 are preferably flush with the guide surfaces 221, 231 or may be recessed from these surfaces. A radiation emitter 251 for emitting infrared radiation, e.g., an infrared diode laser, is arranged in the housing behind the window 228. A radiation detector 252 for detecting infrared radiation is arranged in the housing behind the window 238. The radiation emitter 251 and the radiation detector 252 are illustrated in Fig. 10 in a highly schematic manner only and are to be understood to be not to scale. Further electronic components, such as a microcontroller, one or more circuit boards etc. may be disposed in the hollow interior of the transducer unit 200.

### First embodiment: coupling the transducer unit to the airway adapter

Figures 7 to 12 illustrate how the transducer unit 200 is coupled to the airway adapter 100.

The transducer unit 200 is pushed over the airway adapter 100 along the coupling direction C indicated in Figures 7 and 10, corresponding to the positive x direction, such that the base body 130 of the airway adapter 100 is received in the receiving space 201 of the transducer unit 200. Due to the axially non-symmetric configuration of the base body 130 and transducer unit 200, the airway adapter 100 can be coupled with the transducer unit 200 only in one single well-defined orientation.

During the coupling movement, the guide surfaces 221, 231 of the transducer unit 200 abut against the side surfaces 140, 150 of the airway adapter 100. Thereby, these surfaces define the relative position of the transducer unit and the airway adapter with respect to the lateral direction y. The guide grooves 222, 232 of the transducer unit 200 receive the locking levers 144, 154 of the airway adapter 100. The guide flanges 141, 142, 151, 152 of the airway adapter 100 abut against corresponding front and rear surfaces of the transducer unit 200 and thus facilitate the positioning of the transducer unit 200 on the airway adapter 100 with respect to the longitudinal direction. The transducer unit 200 moves downwards along the coupling direction C until the downwardly facing stop surfaces 226, 236 of the transducer unit 200 abut to the stop flanges 143, 153 of the airway adapter 100 and thus prevent a further downward movement. The resulting coupled state is illustrated in Figures 9 and 12.

As best apparent from Figures 7-9, at some point during the coupling movement, the locking stops 224, 234 of the transducer unit 200 start to come into contact with the inclined sliding surfaces of the locking lugs 145, 155 of the airway adapter 100, pressing the locking lugs 145, 155 gradually inwards. This causes the locking levers 144, 154 to elastically bend inwards towards the center plane P until the locking stops 224, 234 have moved past the locking lugs 145, 155. At this point, the locking levers 144, 154 move outwards again by virtue of the elastic restoring force. The locking lugs 145, 155 are now received in the locking recesses 223, 233, and the transducer unit 200 is prevented from moving upwardly again by a form fit between the locking lugs 145, 155 and the locking stops 224, 234. In this way, the transducer unit 200 is securely held on the airway adapter 100.

In the coupled state, the top surface 131 of the base body 130 of the airway adapter 100 either abuts to the support surface 211 of the transducer unit 200 or has a small distance of not more than 0.2 mm, preferably not more than 0.1 mm from the support surface 211 of the transducer unit 200. The contact pads 171 of the airway adapter 100 compress the spring-loaded contact pins 240 of the transducer unit 200 against their spring force. Thereby, the spring-loaded contact pins 240 exert an upward force on the transducer unit 200, pressing the locking stops 224, 234 against the locking lugs 145, 155. In this manner, undesired play along the x direction is avoided, and a secure electrical connection is established between the airway adapter 100 and the transducer unit 200.

As apparent from Figures 9 and 12, in the coupled state, the side surfaces 140, 150 of the airway adapter 100 are embraced by the lateral wall structures 220, 230 of the transducer unit 200 while the handles 146, 156 remain easily accessible by virtue of the gripping recesses 225, 235.

For releasing the transducer unit 200 from the airway adapter 100, the user presses the handles 146, 156 inwards towards each other along unlocking directions U, U'. Thereby, the locking levers 144, 154 are elastically deflected inwardly towards the center plane P, and the locking lugs 145, 155 are released from the locking recesses 223, 233 such that the locking stops 224, 234 can move upwardly past the locking lugs 145, 155. The transducer unit 200 can now readily be pulled away from the airway adapter 100 along the removal direction R indicated in Fig. 9.

The handles 146, 156 facilitate actuation of the locking levers 144, 154 because they are wider (having larger dimensions along the longitudinal direction z) than the locking levers 144, 154 and because they protrude from the locking levers 144, 154 downwardly and outwardly in a V configuration, providing inclined gripping surfaces with a surface normal that has both a laterally outwardly pointing component and an upwardly pointing component.

### Operation

For operation, the airway adapter 100 is coupled to the transducer unit 200. The radiation emitter 251 is operated to emit infrared radiation 253 (see Fig. 12 for a schematic illustration). The infrared radiation 253 passes through the aperture 227 and window 228 of the transducer unit 200 and through the aperture 147 and window 148 of the airway adapter 100 before it enters the sampling passage 160, where it is attenuated by the gas contained in the sampling passage 160. The remaining infrared radiation leaves the sampling passage 160 through the window 158 and aperture 157 of the airway adapter 100 and through the window 238 and aperture 237 of the transducer unit 200 before it impinges onto the radiation detector 252. Based on the signals of the radiation detector 252, the concentration of carbon dioxide in the gas in the sampling passage 160 is determined. Additional information about the gas may be determined using the sensor 170.

### Second embodiment: dual locking levers on each side of base body

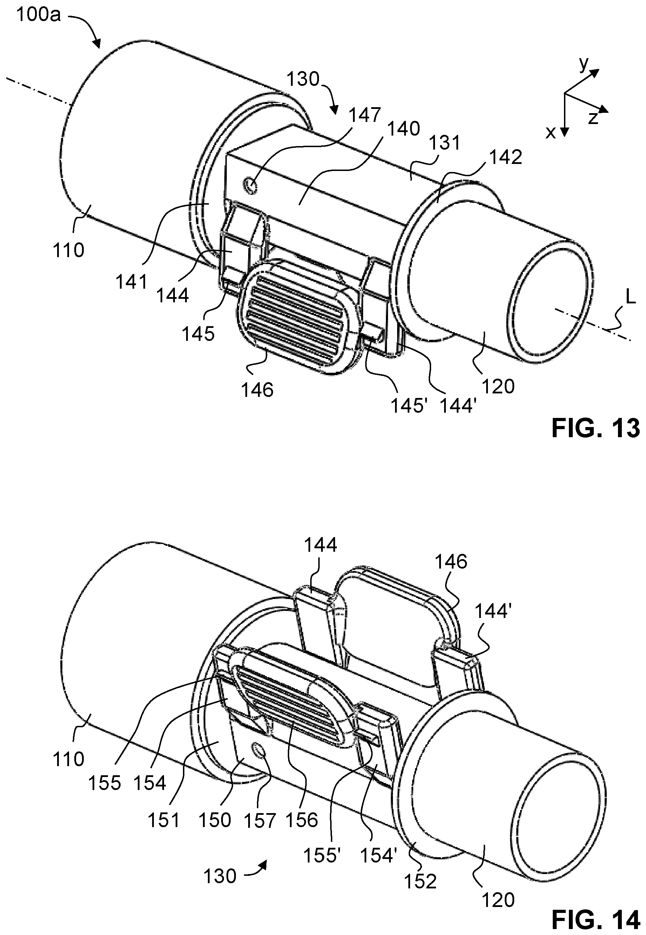

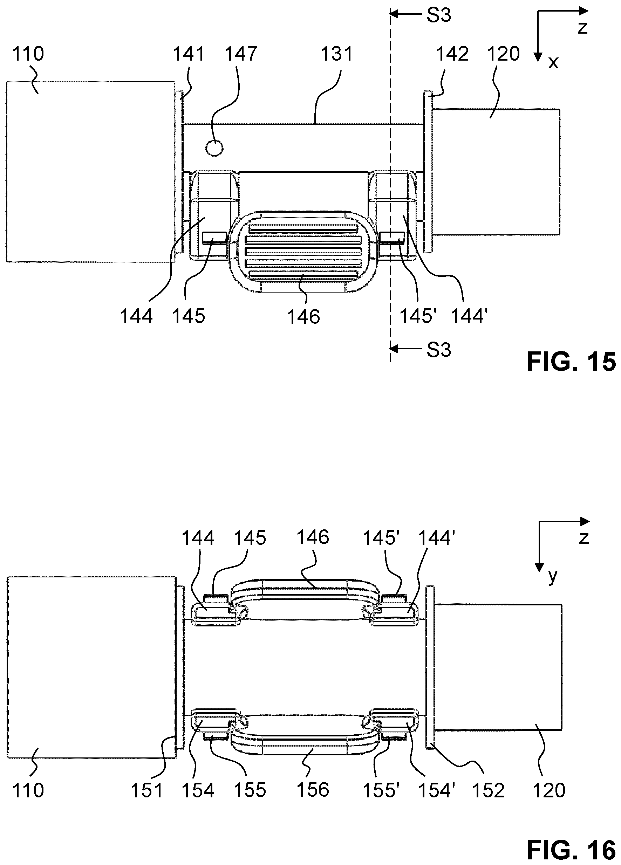

An airway adapter 100a and a corresponding transducer unit 200a according to a second embodiment are illustrated in Figs. 13 to 18. Corresponding elements are provided with the same reference signs as for the first embodiment, and a description thereof will be omitted.

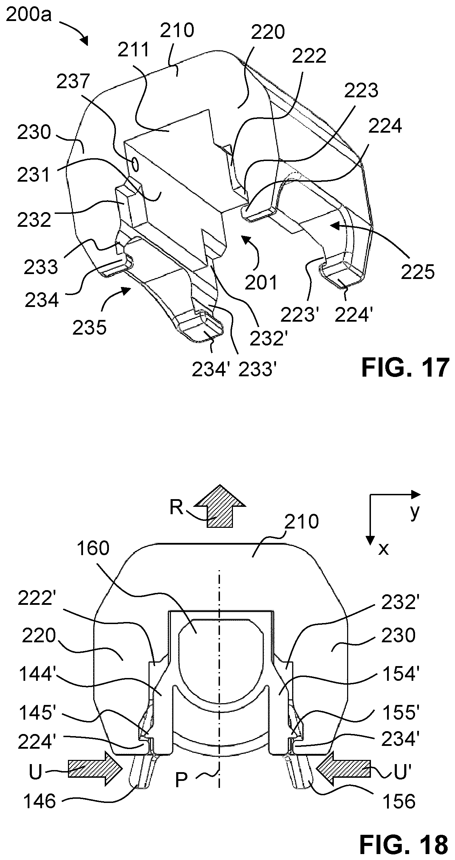

As in the first embodiment, the airway adapter 100a comprises a base body 130 comprising side surfaces 140, 150. On one lateral side of the base body 130, the airway adapter 100a comprises two first locking levers 144, 144' in the form of spring arms, which are spaced along the longitudinal direction z. An outwardly projecting first locking lug 145, 145' is formed on each first locking lever 144, 144'. A common first handle 146 bridges the free distal ends of the first locking levers 144, 144'. The handle 146 has an inclined gripping surface at its outside. The gripping surface is provided with a plurality of parallel ribs extending longitudinally to improve grip. In a symmetric manner, two second locking levers 154, 154', second locking lugs 155, 155' and a second handle 156 are arranged at the opposite lateral side of the base body 130 with respect to the center plane P indicated in Fig. 18.

As in the first embodiment, the transducer unit 200a has a base 210 and two mutually opposing lateral wall structures 220, 230 delimiting a receiving space 201 for receiving the airway adapter 100a between them. In each lateral wall structure, two vertical guide grooves 222, 222' and 232, 232', respectively are formed to receive the locking levers 144, 144', 154, 154' of the airway adapter 100'. In the present example, the guide grooves are not only open downwardly in the positive x direction but also open towards the respective longitudinal end of the transducer unit 200' and thus may also be referred to as guide recesses. Below each guide groove, a locking recess 223, 223', 233, 233' is formed. Each locking recess is delimited downwardly by a locking stop 224, 224', 234, 234'. The locking recesses and locking stops together form locking hooks on the transducer unit 200a.

In contrast to the first embodiment, the airway adapter 100a does not comprise a gas sensor, and no contact pads are provided on the airway adapter 100a. The airway adapter 100a further lacks the stop flanges 143, 153 of the first embodiment, and the transducer unit 200a lacks the stop surfaces 226, 236 of the first embodiment. Accordingly, when the transducer unit 200a is pushed over the airway adapter 100a, it moves downwardly until the support surface 211 of the transducer unit 200a abuts to the top surface 131 of the base body 130. In this state, the locking lugs 145, 145', 155, 155' are received in the locking recesses 223, 223', 233, 233' of the transducer unit 200a, and the transducer unit 200a is prevented from moving upwardly by the locking stops 224, 224, 234, 234' interacting with the locking lugs 145, 145', 155, 155'. For releasing the airway adapter 100a, the handles 146, 156 are pressed laterally towards each other in directions U, U' to release the locking lugs from the locking recesses, and the transducer unit 200a is pulled away from the airway adapter 100a in the removal direction R, as in the first embodiment.

### Third embodiment: protruding windows

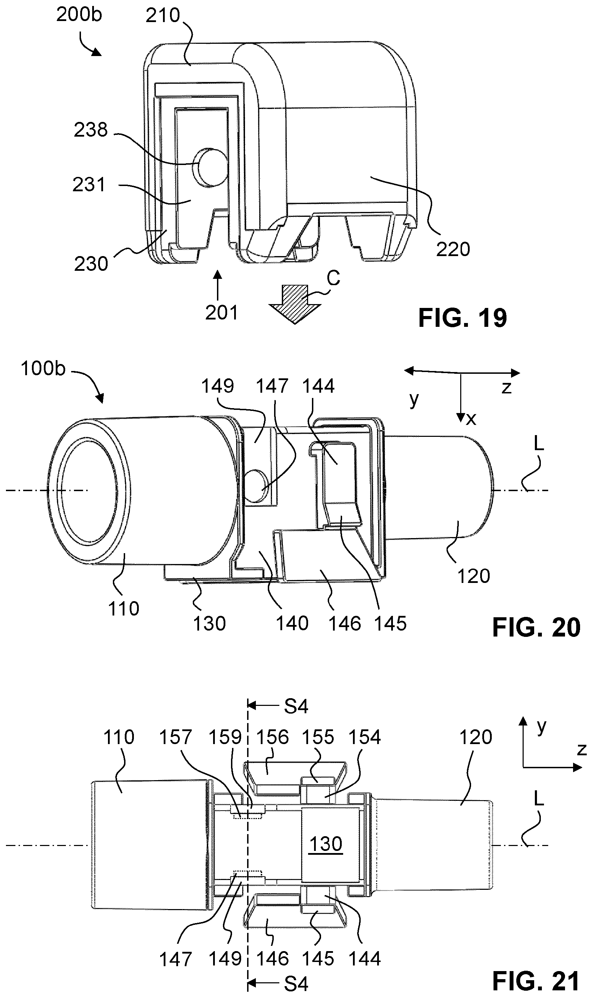

An airway adapter 100b and a corresponding transducer unit 200b according to a third embodiment are illustrated in Figs. 19 to 22.

In contrast to the first and second embodiments, the optical windows 228, 238 of the transducer unit 200 protrude inwardly from the guide surfaces 221, 231 into the receiving space 201. The protruding portion of each optical window 228, 229 is cylindrical, having a cylinder axis along the lateral direction y.

In order to avoid a collision with the protruding window portions when the transducer unit 200b is coupled with the airway adapter 100b, protrusion-receiving grooves 149, 159 are formed in the side surfaces 140, 150 of the airway adapter 100b. Each of the protrusion-receiving grooves 149, 159 extends in the coupling direction C and is a blind groove, extending between an open end located at a peripheral boundary of the respective side surface 140, 150 and a closed end positioned within the respective side surface. Each of the apertures 147, 157 of the airway adapter 100b extends between one of the protrusion-receiving grooves 149, 159 and the sampling passage 160. Accordingly, each of the windows 148, 158 of the airway adapter 100b is arranged within the corresponding protrusion-receiving groove 149, 159.

Expressed differently, each of the protrusion-receiving grooves 149, 159 is a recessed slot or channel formed in the respective side surface 140, 150, and the apertures 147, 157 with their associated windows 148, 158 are arranged within the slot or channel at its bottom. Being a blind groove, each protrusion-receiving groove does not extend fully across the respective side surface. In the present example, each protrusion-receiving groove 149, 159 has rectangular cross section; however, as will be shown below, different cross sectional shapes are also possible.

The open end of each of the protrusion-receiving grooves 149, 159 is open in the negative x direction, i.e., in a direction opposite to the coupling direction C, to enable the corresponding protruding window portion to be received in the protrusion-receiving groove when the transducer unit 200b is coupled with the airway adapter 100b. Each protrusion-receiving groove 149, 159 has a width that essentially corresponds to the outer dimension of the corresponding protruding window portion along the longitudinal direction z. Each protrusion-receiving groove has a considerable depth, measured along the lateral direction, which in the present example corresponds to slightly more than the remaining wall thickness between the protrusion-receiving groove and the sampling passage.

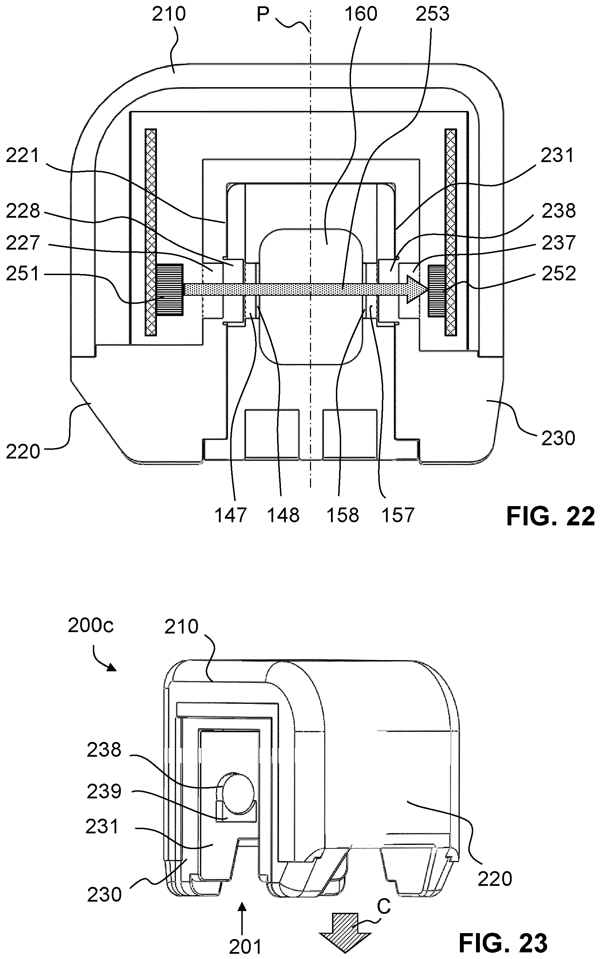

By providing the transducer unit 200 with inwardly protruding windows 228, 238, the distance between the windows 228, 238 of the transducer unit 200b and the corresponding windows 148, 158 of the airway adapter 100b is reduced. This is readily apparent by comparing said distance in Fig. 22 to the corresponding distance in Fig. 12. Nevertheless, mechanical stability of the airway adapter 100b is not compromised because the wall thickness of the airway adapter 100b remains the same as in the previous embodiments except for the (rather small) region of the protrusion-receiving grooves 149, 159.

By reducing the distance between the windows 228, 238 of the transducer unit 200b and the corresponding windows 148, 158 of the airway adapter 100b, the dead space between these windows is reduced. This dead space can get filled with a CO₂-containing gas during coupling, which may cause measurement errors when the CO₂ concentration of a gas in the sampling passage is determined. Reducing the dead space thus improves reliability and accuracy of the measurements that are carried out with the gas analyzer.

Stability of the airway adapter is improved by keeping the diameter of the windows 148, 158 and the width of the protrusion-receiving grooves 149, 159 of the airway adapter small. For example, the diameter of the windows 148, 158 and/or the width of the protrusion-receiving grooves may be only about 2 mm.

In order to provide infrared radiation of sufficient intensity despite such a small diameter, the radiation source 251 is preferably an infrared laser, which provides an intense infrared beam with small divergence.

### Fourth embodiment: Window support

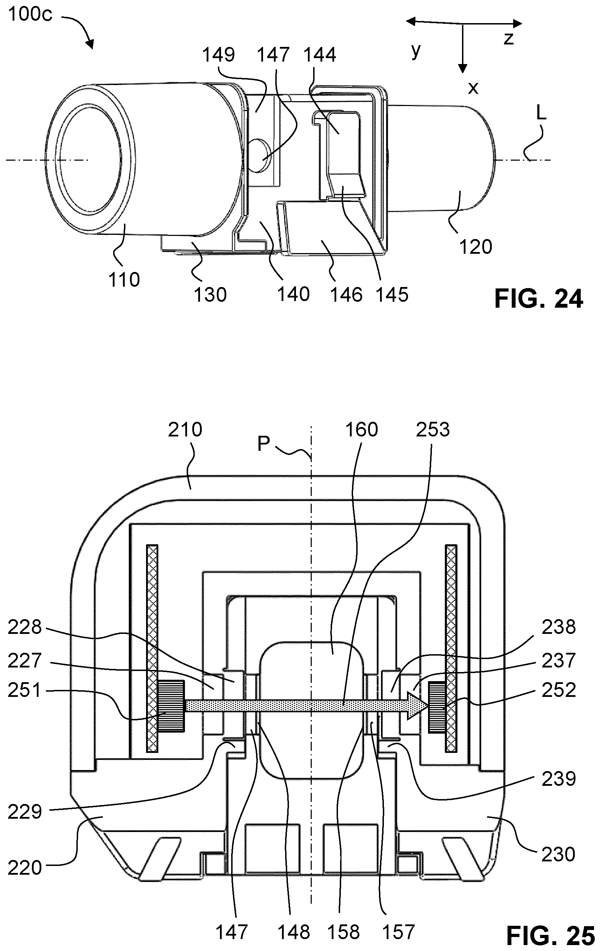

An airway adapter 100c and a corresponding transducer unit 200c according to a fourth embodiment are illustrated in Figs. 23 to 25.

In this embodiment, each of the protruding windows 228, 238 of the transducer unit 200c is mechanically supported and protected by a window support 229, 239. Each window support 229, 239 protrudes inwardly from the respective guide surface 221, 231 and is arranged just below the respective window 228, 238 along the coupling direction C. Each window support protrudes inwardly from the respective guide surface 221, 231 at least by the same amount as the associated window. Each window support is formed integrally (in one piece) with the associated guide surface. Each window support has a dimension along the longitudinal direction that at least corresponds to the maximum dimension of the associated protruding window portion along this direction.

In order to accommodate the window supports 229, 239, the protrusion-receiving grooves 149, 159 of airway adapter 100c are slightly longer along the x direction than in airway adapter 100b.

The window supports 229, 239 protect the protruding portions of the windows 228, 238 from damage during the coupling movement. This may in particular relevant if a user tries to couple the transducer unit 200c with an incompatible airway adapter without a protrusion-receiving groove, such as the airway adapter of the first embodiment.

### Fifth and sixth embodiments: Convex window

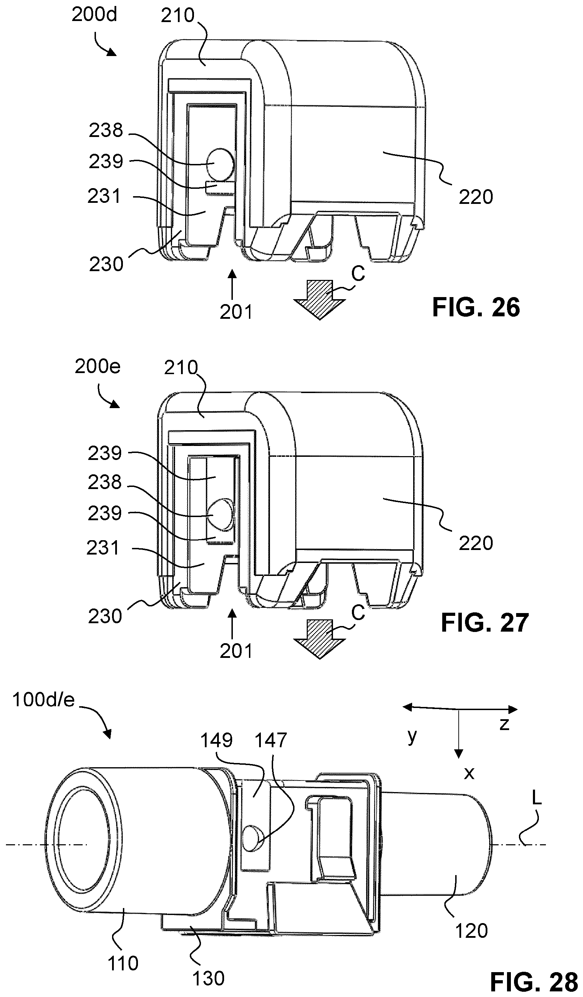

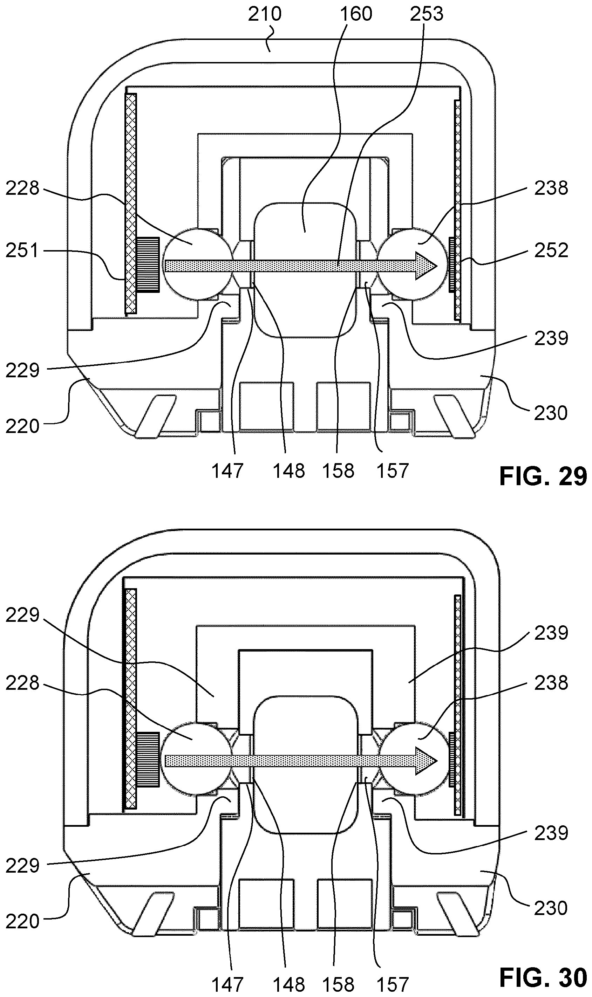

A transducer unit 200d according to a fifth embodiment and a transducer unit 200e according to a sixth embodiment are illustrated in Figs. 26 and 27. A corresponding airway adapter 100d/e is illustrated in Fig. 28. Figure 29 illustrates the airway adapter 100d/e and the transducer unit 200d in a coupled state. Figure 30 illustrates the airway adapter 100d/e and the transducer unit 200e in a coupled state.

In these embodiments, the portions of the windows 228, 238 that protrude into the receiving space 201 have a convexly curved shape. In particular, the protruding portions are convexly curved with respect to both the x and z directions. The windows 228, 238 thus form converging lenses for shaping the infrared radiation. More specifically, each window 228, 238 is spherical (ball-shaped), being formed by a sphere made of an infrared-transparent material. However, instead of being spherical, the windows 228, 238 may have another suitable shape that convexly protrudes into the receiving space 201, e.g., each window may form an ellipsoid or may have a cylindrical portion adjoined by a convexly curved portion.

In the transducer unit 200d, the windows 228, 229 are protected by a window support 229, 239 arranged just below the respective window 228, 238 along the coupling direction C, similar to the window supports of transducer unit 200c. In contrast to transducer unit 200c, the window support has an arcuate shape matching the curvature of the curved protruding window portions, similar to a segment of a flat circular disk whose disk axis extends parallel to the coupling direction C.

In the transducer unit 200e, the window supports 229, 239 are not only arranged just below the respective window 228, 238 along the coupling direction C, but also extend above the respective window essentially all the way between the base 210 and the respective window 228, 238 to provide additional mechanical strength to the sidewall portions 220, 230 in the region of the guide surfaces 221, 231. The window support has the shape of a segment of a right circular cylinder whose cylinder axis extends parallel to the coupling direction C.

In order to match the curved shape of the windows 228, 238 and window supports 229, 239 of the transducer units 200d, 200e, the protrusion-receiving grooves 149, 159 of the airway adapter 100d/e are trough-shaped, having a concave cross section instead of a rectangular cross section as in the airway adapters 100b and 100c.

### Seventh embodiment: Flow and pressure sensors

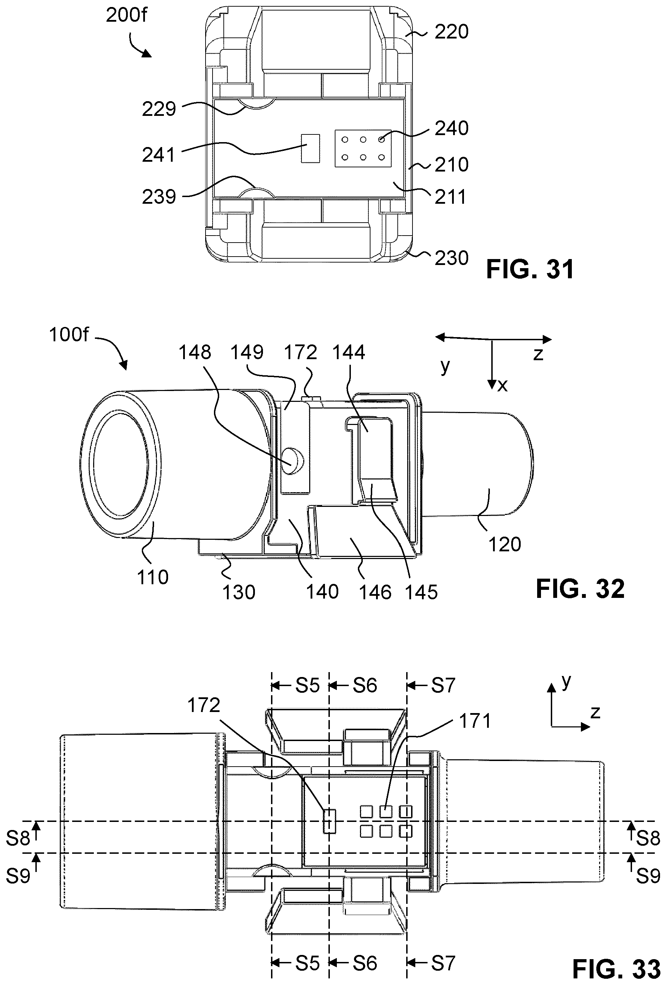

A transducer unit 200f and a corresponding airway adapter 100f according to a seventh embodiment are illustrated in Figs. 31 to 40.

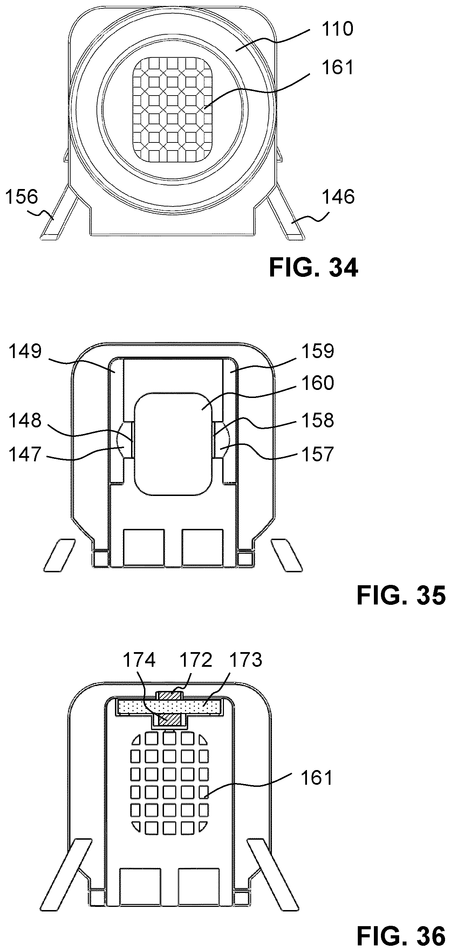

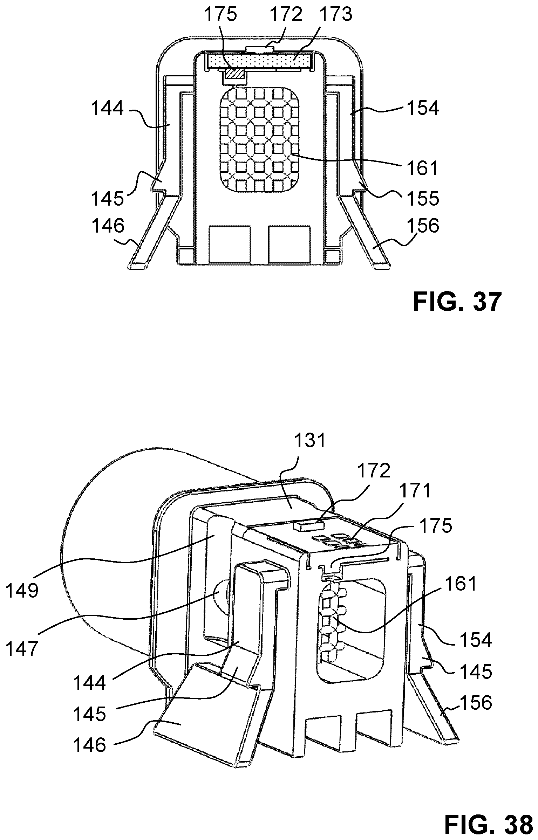

The airway adapter 100f comprises a flow restrictor 161 (see Figs. 34 and 36-40), contact pads 171 (see Figs. 33 and 38), a heating resistor 172 (see Figs. 32, 33 and 36-40), a circuit board 173 (see Figs. 36-40), a flow rate sensor 174 (see Figs. 36, 39 and 40) and a pressure sensor 175 (see Figs. 37-40).

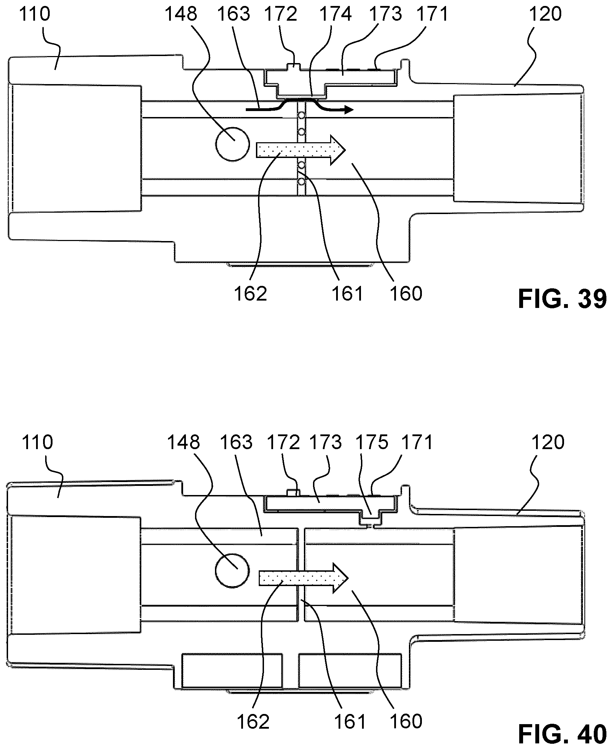

The flow restrictor 161 extends across the sampling passage 160, separating an upstream side of the sampling passage 160 from a downstream side of the sampling passage 160 when a main gas flow 162 (see Fig. 39) passes through the sampling passage 160 along the positive z direction. A narrow bypass channel bypasses the flow restrictor 161. The flow restrictor 161 forces part of the gas in the sampling passage 160 to flow through the bypass channel as a bypass flow 163.

The flow rate sensor 174 is exposed to the bypass flow 163 through a vertical opening in a wall of the airway adapter 100f that delimits the bypass channel. The flow rate sensor 174 may be a microthermal flow rate sensor comprising a heater element and two temperature sensors upstream and downstream of the heater element, as it is well-known in the art. The heater element is heated by supplying a current to it, and a flow rate through the bypass channel is determined based on the resulting temperature difference between the upstream and downstream temperature sensors. An example of a suitable flow rate sensor is provided in WO 01/98736 A1. Many other examples exist, and the present disclosure is not limited to a particular type of flow rate sensor.

The pressure sensor 175 is exposed to the gas in the sampling passage 160 through a small vertical opening in a wall of the airway adapter 100f that delimits the sampling passage 160. The pressure sensor 175 is arranged slightly laterally offset from the center plane P. It determines the static pressure in the sampling passage 160. In addition or in the alternative, a different type of sensor may be provided, e.g., a temperature sensor, a humidity sensor or another type of concentration sensor.

The heating resistor 172 may be supplied with a current to heat the airway adapter 100f to reduce or prevent condensation of liquid water from the gas in the sampling passage. In particular, condensation in the narrow bypass channel and on the flow rate sensor 175 can be efficiently reduced or prevented in this manner.

The flow rate sensor 174, the pressure sensor 175 and the heating resistor 172 are mounted on the circuit board (PCB) 173. Also provided on the circuit board are the contact pads 171. The contact pads 171 cooperate with the flow rate sensor 174, the pressure sensor 175 and the heating resistor 172 to supply electric currents to these elements and to read out a flow rate signal from the flow sensor 174 and a pressure signal from the pressure sensor 175.

The flow rate sensor 174 and the pressure sensor 175 are sealed against the housing of the airway adapter 101f by a suitable sealant, e.g., by an adhesive, which may e.g. be epoxy- or silicone-based.

Of course, the sensors can be arranged differently than illustrated, and different types of sensors can be used.

### Eighth embodiment: Inclined beam path

In the above-discussed exemplary embodiments, the radiation emitter 251 and the radiation detector 252 are aligned along the y direction, which is perpendicular both to the longitudinal direction z and the coupling direction C (or, equivalently, the x direction). Accordingly, the windows 148, 158 of the airway adapter and the windows 228, 238 of the corresponding transducer unit are also aligned along the y direction. An infrared light ray that is emitted from the center of the radiation emitter 251 travels to the center of the radiation detector 252 along an optical axis that runs along the y direction.

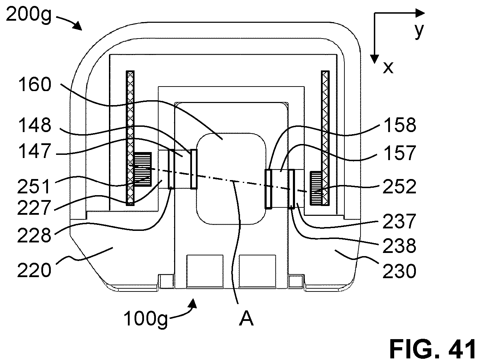

However, the optical axis, while preferably still being perpendicular to the longitudinal direction z, may be inclined relative to the y direction by a non-zero angle, having components along both the y direction and the x direction. An embodiment having an inclined optical axis A is illustrated by the way of example in Fig. 41, which illustrates an airway adapter 100g and a corresponding transducer unit 200g according to an eighth embodiment in their coupled state. In this embodiment, the radiation emitter 251 is offset from the radiation detector 252 with respect to the x direction such that an infrared light ray that is emitted from the center of the radiation emitter 251 travels to the center of the radiation detector 252 along the inclined optical axis A. Accordingly, the window 228 of the transducer unit 200g is offset from the window 238 with respect to the x direction, and the window 148 of the airway adapter 100g is also offset from the window 158 with respect to the x direction. The optical axis A is inclined relative to the y direction by a non-zero angle, which in the present embodiment is about 8°. Like in the previously discussed embodiments, the optical axis A is perpendicular to the z direction.

An arrangement with an inclined optical axis may be advantageous because some laser light sources have an emission profile that is not fully orthogonal to a mounting surface of the light source but may have a slight angle of up to 20°, e.g., due to production restrictions. While a fully orthogonal emission profile may be possible, it may come with increased production cost. Providing the gas analyzer with an inclined optical axis may therefore be more cost-effective than having a horizontal optical axis.

An inclined optical axis may also be provided in embodiments having a protruding and/or convex window, as described in more detail above.

### Modifications

The present disclosure is not limited to the above embodiments, and many modifications are possible.

In particular, the features of the various embodiments described herein may be present in isolation or may be combined. For example, an airway adapter having two first locking levers bridged by a first handle and two second locking levers bridged by a second handle, as in the second embodiment, can also be provided with an additional sensor as in the first and seventh embodiments. A corresponding transducer unit may also have protruding windows as in the third to sixths embodiments. Protruding windows may also be provided if the airway adapter does not have locking levers.

In the exemplary embodiments illustrated in the drawings, the end sections 110, 120 of the airway adapter are configured as pipes that slightly taper towards their free ends ("medical cones"), enabling one end of the airway adapter to be connected to an endotracheal tube or to a breathing mask and the other end to a mechanical ventilator or to a self-inflating bag of a "bag valve mask (BVM)" type manual resuscitator. However, the end sections may have arbitrary shape depending on the intended use of the airway adapter. For example, one of the end sections may be configured to connect to a patient's nostrils, as it is known per se from the prior art. The end sections do not need to be axially aligned with the sampling passage 160 along a common centerline, as in the above-described exemplary embodiments. For example, the end sections may extend parallel but slightly offset to the centerline.

Also, the base body 130 may be shaped differently than illustrated. In particular, the side surfaces 140, 150 do not need to be planar and parallel. For example, the base body 130 may taper upwardly, the distance between the side surfaces decreasing upwardly along the negative x direction, and the receiving space 201 of the transducer unit 200 may taper upwardly in a similar manner to facilitate insertion of the base body 130 into the receiving space 201. The guide flanges 141, 142, 151, 152 of the first embodiments, while useful for positioning the base body 130 in the transducer unit with respect to the longitudinal direction, are not necessary and can be dispensed with. Likewise, the stop flanges 143, 153 of the first embodiment, while being useful for defining the end position of the transducer unit with respect to the coupling direction C, are not required and can be dispensed with. In this case, the end position will be defined by the support surface 211 of the transducer unit abutting to the top surface 131 of the base body 130.

Instead of configuring the locking levers as spring arms that can bend elastically, it is also conceivable to provide rigid locking arms that are connected to the base body 130 via a hinge (e.g., via a film hinge) and to bias the locking arms outwardly away from the base body 130 by a separate spring, e.g., by a helical spring or by a leaf spring.

Instead of providing one or more pairs of mutually opposing locking levers, as illustrated, it is also possible to provide only one single locking lever on one lateral side of the base body 130, the single locking lever carrying a handle as described above. To facilitate removal of the airway adapter from the transducer unit, a second handle may be provided in an opposing manner to the handle on the single locking lever with respect to the center plane, the second handle being immovable relative to the base body 130.

Instead of a locking lug, any kind of locking structure may be provided on the locking lever. For example, a locking recess may be provided on the outside of the locking lever, the locking recess cooperating with a locking stop on the transducer unit 200 in the form of a locking lug. Many other shapes of the locking structure are possible.

### LIST OF REFERENCE SIGNS

- 100, 100a-100g: airway adapter
- 110: first end section
- 120: second end section
- 130: base body
- 131: top surface
- 140: side surface
- 141: guide flange
- 142: guide flange
- 143: stop flange
- 144, 144': locking lever
- 145, 145': locking lug
- 146: handle
- 147: aperture
- 148: window
- 149: protrusion-receiving groove
- 150: side surface
- 151: guide flange
- 152: guide flange
- 153: stop flange
- 154, 154': locking lever
- 155, 155': locking lug
- 156: handle
- 157: aperture
- 158: window
- 159: protrusion-receiving groove
- 160: sampling passage
- 161: flow resistor
- 162: main gas flow
- 163: bypass flow
- 170: electrically operated sensor
- 171: contact pads
- 172: heater element
- 173: circuit board / PCB
- 174: flow rate sensor
- 175: pressure sensor
- 200, 200a-200g: transducer unit
- 201: receiving space
- 210: base
- 211: support surface
- 220: lateral wall structure
- 221: guide surface
- 222: guide groove
- 223: locking recess
- 224: locking stop
- 225: gripping recess
- 226: stop surface
- 227: aperture
- 228: window
- 229: window support
- 230: lateral wall structure
- 231: guide surface
- 232: guide groove
- 233: locking recess
- 234: locking stop
- 235: gripping recess
- 236: stop surfaces
- 237: aperture
- 238: window
- 239: window support
- 240: contact pins
- 241: recess
- 251: radiation emitter
- 252: radiation detector
- 253: infrared radiation
- A: optical axis
- C: coupling direction
- L: centerline
- P: center plane
- R: removal direction
- U, U': unlocking direction

## Claims

1. A transducer unit for a gas analyzer, comprising:
a housing comprising a base (210) and first and second lateral wall structures (220, 230) extending away from the base (210) in a coupling direction (C) and being arranged at a distance to each other with respect to a lateral direction (y) that is transverse to the coupling direction (C), the first and second lateral wall structures (220, 230) defining a receiving space (201) between them for receiving an airway adapter, the receiving space (201) being open in the coupling direction (C);
a radiation emitter (251); and
a radiation detector (252),
wherein the first lateral wall structure (220) has a first guide surface (221) that faces the receiving space (201),
wherein a first optical window (228) is provided in the first lateral wall structure (220) at the first guide surface (221),
wherein the radiation emitter (251) is received in the housing to emit infrared radiation (253) towards the receiving space (201) through the first optical window (228),
wherein the second lateral wall structure (220) has a second guide surface (231) that faces the receiving space (201),
wherein a second optical window (238) is provided in the second lateral wall structure (230) at the second guide surface (231),
wherein the radiation detector (252) is received in the housing to detect a portion of the infrared radiation (253) that has traversed the receiving space (201) and the second optical window (238),
**characterized in that**
at least one of the first and second optical windows (228, 238) has a protruding window portion that protrudes from the first or second guide surface (221, 231), respectively, inwardly towards the receiving space (201).

2. The transducer unit of claim 1, wherein the protruding window portion is convexly curved.

3. The transducer unit of claim 2,
wherein the protruding window portion is convexly curved both along the coupling direction (C) and along a longitudinal direction (z) that extends transversely to both the coupling direction (C) and the lateral direction (y),
in particular, wherein the optical window (228, 238) having the protruding window portion is spherical.

4. The transducer unit of any one of the preceding claims,
further comprising a window support (229, 239) arranged adjacent to the protruding window portion with respect to the coupling direction (C), the window support (229, 239) protruding inwardly from the first or second guide surface (221, 231) towards the receiving space (201) at least by the same amount as the protruding window portion.

5. The transducer unit of claim 4, wherein the window support (229, 239) has a longitudinal dimension, measured along a longitudinal direction (z), that at least corresponds to a maximum longitudinal dimension of the protruding window portion, measured along the longitudinal direction (z), the longitudinal direction (z) being transverse to both the coupling direction (C) and the lateral direction (y).

6. The transducer unit of claim 4 or 5, wherein the window support (229, 239) extends on two sides of the protruding window portion with respect to the coupling direction (C).

7. An airway adapter comprising:
a base body (130) defining a sampling passage (160) for a gas to be analyzed, the sampling passage (160) extending through the base body (130) along a longitudinal direction (z), the base body (130) having a first side surface (140) on a first lateral side of the base body (130) and a second side surface (150) on an opposing second lateral side of the base body (130) with respect to a center plane (P) that contains the longitudinal direction (z) and passes centrally through the sampling passage (160);
a first optical window (148) at the first lateral side of the base body (130) and a second optical window (158) at the second lateral side of the base body (130) to allow infrared radiation (253) to enter and exit the sampling passage (160) transversely to the longitudinal direction (z);
**characterized in that**
the base body (130) has a first protrusion-receiving groove (149) formed in the first side surface (140) of the base body (130), the first protrusion-receiving groove (149) extending in a coupling direction (C) that extends transversely both to the longitudinal direction (z) and to a lateral direction (y), the lateral direction (y) being perpendicular to the center plane (P), the first protrusion-receiving groove (149) being a blind groove extending between an open end located at a peripheral boundary of the first side surface (140) and a closed end positioned within the first side surface (140), and
the first optical window (148) is arranged in the first protrusion-receiving groove (149).

8. The airway adapter of claim 7, wherein a width of the first protrusion-receiving groove (149), measured along the longitudinal direction (z), is no greater than 1.5 times a diameter of the first optical window (148).

9. The airway adapter of claim 7 or 8, wherein a length of the first protrusion-receiving groove (149), measured along the coupling direction (C), is at least 1.5 times a diameter of the first optical window (148).

10. The airway adapter of any one of claims 7 to 9, wherein a depth of the first protrusion-receiving groove (149), measured along the lateral direction (y), is at least 50% of a remaining wall thickness between the first protrusion-receiving groove (149) and the sampling passage (160), measured along the lateral direction (y).

11. The airway adapter of any one of claims 7 to 10,
wherein the base body (130) has a second protrusion-receiving groove (159) formed in the second side surface (150) of the base body (130), the second protrusion-receiving groove (159) extending parallel to the first protrusion-receiving groove (149), the second groove (159) being a blind groove extending between an open end located at a peripheral boundary of the second side surface (150) and a closed end positioned within the second side surface (150), and
the second optical window (158) is arranged in the second protrusion-receiving groove (159).

12. The airway adapter (100) of any one of claims 7 to 11, wherein the first and second optical windows (148, 158) are offset relative to each other in the coupling direction (C).

13. A gas analyzer comprising:
the transducer unit of any one of claims 1 to 6; and
the airway adapter of any one of claims 7 to 12,
wherein the transducer unit is configured to be coupled to the airway adapter along the coupling direction (C),
wherein the first protrusion-receiving groove (149) receives the protruding window portion of the transducer unit when the transducer unit is coupled to the airway adapter.

14. The gas analyzer of claim 13, wherein a width of the first protrusion-receiving groove (149), measured along the longitudinal direction (z), corresponds to an outer dimension of the protruding window portion or of an associated window support of the transducer unit, measured along the longitudinal direction (z).

15. The gas analyzer of claim 13 or 14, wherein the first guide surface (221) abuts against the first side surface (140) and the second guide surface (231) abuts against the second side surface (150) when the transducer unit is coupled to the airway adapter.
